# EUROPEAN PATENT SPECIFICATION

(11) **EP 1 460 130 B1**
(45) Date of publication and mention of the grant of the patent: **21.03.2007**
(21) Application number: 01275023.8
(22) Date of filing: 19.12.2001
(51) Int. Cl.: C12N 15/09, C12Q 1/68, C12M 1/00, H01L 29/06, H01L 29/66

(54) **POTENTIOMETRIC DNA MICROARRAY, PROCESS FOR PRODUCING THE SAME AND METHOD OF ANALYZING NUCLEIC ACID**
POTENTIOMETRISCHER DNA-MIKROARRAY, VERFAHREN ZU DESSEN HERSTELLUNG UND VERFAHREN ZUR NUKLEINSÄUREANALYSE
MICRORESEAU D'ADN POTENTIOMETRIQUE, PROCEDE DE FABRICATION CORRESPONDANT ET PROCEDE D'ANALYSE D'ACIDE NUCLEIQUE

(43) Date of publication of application: 22.09.2004
(73) Proprietor: Hitachi High-Technologies Corporation, Tokyo 105-8717 (JP)
(72) Inventor: MIYAHARA, Yuji c/o Hitachi High-Technologies Corp., Hitachinaka-shi, Ibaraki 312-8504 (JP); YASUDA, Kenji, c/o Hitachi High-Technologies Corp., Hitachinaka-shi, Ibaraki 312-8504 (JP); HATTORI, Kumiko, c/o Hitachi High-Technolog. Corp., Hitachinaka-shi, Ibaraki 312-8504 (JP)
(74) Representative: Strehl Schübel-Hopf & Partner
(86) International application number: PCT/JP2001/011150
(87) International publication number: WO 2003/052097

(56) References cited:
- EP-A1- 0 882 979
- WO-A-02/086162
- WO-A-03/014722
- US-A- 5 827 482
- YUJI MIYAHARA: 'Microfabrication-gijutsu o mochiiru biosensing system' CHROMATOGRAPHY vol. 22, May 2001, pages 83 - 84, XP002951583
- YI CUI ET AL.: 'Nanowire nanosensors for highly sensitive and selective detection of biological and chemical species' SCIENCE vol. 293, no. 5533, August 2001, pages 1289 - 1292, XP002951584
- GRAHAM RAMSAY ET AL.: 'DNA chips: State-of-the art' NATURE BIOTECHNOLOGY vol. 16, no. 1, 1998, pages 40 - 44, XP002951585
- AMEUR S. ET AL.: 'Immunosensing using electrochemical impedance spectroscopy on functionalized gold electrodes' CHEMICAL AND BIOLOGICAL SENSORS AND ANALYTICAL ELECTROCHEMICAL METHODS 1997, pages 1019 - 1023, XP002951586

### SEQUENCE LISTING

<110> HITACHI HIGH-TECHNOLOGIES CORPORATION
<120> Potentiometric DNA microarray, process for producing the same and method of analyzing nucleic acid
<130> PH-1266-PCT
<140> PCT/JP01/11150
   <141> 2001-12-19
<160> 2
<170> Patent In Ver. 2.1
<210> 1
   <211> 17
   <212> DNA
   <213> Artificial Sequence
<220>
   <223> Description of Artificial Sequence:probe
<400> 1
   catacactaa agtgaaa 17
<210> 2
   <211> 17
<212> DNA
   <213> Artificial Sequence
<220>
   <223> Description of Artificial Sequence:probe
<400> 2
   catacactga agtgaaa 17

## Description

### TECHNICAL FIELD

The present invention relates to biotechnology in such field as genetic diagnosis, sequence analysis of DNA, or analysis of single nucleotide polymorphism, particularly to technology in the field of genetic testing and more specifically, to potentiometric DNA microarray capable of simultaneously analyzing a plurality of different nucleic acids with high accuracy, a process for producing the microarray and a method of analyzing nucleic acids.

### BACKGROUND ART

Rapid progress has been made in the projects of genome nucleotide sequence analysis for various living organisms including the human genome project, and enormous amounts of information on the nucleotide sequence are being accumulated. At present, the entire nucleotide sequence of the human genome is being determined. From now on, elucidation of gene functions in vivo seems likely to promote dramatic developments of gene-related technology in a wide range of fields including diagnosis of various diseases, pharmaceutical development, breeding of agricultural products, and the like. The foundation for the progress in these new fields is formed by information about gene expression and function in addition to information on nucleotide sequences. As a technology to conduct the analysis of gene function and gene expression in a large scale and to develop it to genetic testing, DNA chip or DNA microarray (hereinafter, collectively called DNA microarray) has been developed by Affymetrix Inc., Nanogen Inc., and so on. Since the majority of the present DNA microarrays utilize detection by fluorescence as the basic principle, a laser or a complex optical system is required for them, and the system becomes larger in size and expensive.

Furthermore, all DNA microarrays developed currently are discarded after only a single use as a general rule. Even if these DNA microarrays may be used repeatedly by washing, their use is limited to at most two to three times, thus giving rise to a major problem of the running cost in analyzing many samples and in the fields of gene diagnosis to test a large number of samples and the like. Particularly in the field of medicine, it is difficult for an expensive test to come into wide use in view of cost containment of medical expenses. On the other hand, high accuracy and quantitative determination are required in the field of the medicine, that is, in the field of gene diagnosis. Accordingly, a technology to satisfy both cost reduction and high accuracy is sought.

For a method to solve these problems, several DNA microarrays having a current detection system combined with oxidation-reduction labeling have been reported. Clinical Micro Sensor Systems, Inc. has developed a system in which one end of a molecule called molecular wire is immobilized on a metal electrode while the other end is bound to a nucleic acid probe, and donation and acceptance of electrons between an oxidation-reduction label and the metal electrode arising from hybridization of the nucleic acid probe with the target gene is detected as a change of electric current to detect target genes (Nature Biotechnology, vol. 16 (1998) p.27, p.40). Since this system does not require an expensive laser or a complex optical system, a simple and small system can be constructed. It utilizes, however, an oxidation-reduction reaction on the metal electrode as the basic principle of the detection, and therefore, the presence of an oxidizing substance or a reducing substance (e.g., ascorbic acid) in a sample induces an electric current due to its oxidation or reduction, thereby disturbing the gene detection and deteriorating accuracy of the detection. Moreover, an electrode reaction occurs progressively on the metal electrode concurrently with the current measurement. Since the electrode reaction is irreversible and is a nonequilibrium reaction, corrosion of the electrode, evolution of gas, and the like occur, resulting in instability of the current measurement and deterioration of detection accuracy particularly when the measurement is repeated.

From these backgrounds, an object of the present invention is to provide a DNA microarray that allows measurement of high accuracy using a system having a low running cost and a low price, a process for producing the DNA microarray, and a method of analyzing nucleic acids with the use of the DNA microarray.

### DISCLOSURE OF THE INVENTION

The present invention is a DNA microarray in which a nucleic acid probe is immobilized on the surface of an insulator and then hybridized with a target gene on the surface of the insulator, and the resulting change in the electric charge density is detected. The DNA microarray is preferably the one including a system in which a nucleic acid probe is immobilized on the surface of a gate insulator of electric field effect transistor and then hybridized with a target gene on the surface of the gate insulator, and the resulting change in the electric charge density is detected by utilizing a field effect. A DNA microarray that allows a potentiometric detection of a change in the surface electric potential with a high signal to noise ratio could be realized by introducing an intercalator or by labeling nucleic acids with a molecule to form a complex with a charged particle such as ion in order to amplify the change in the surface electric charge density in addition to the charge inherent in the nucleic acids. Since a method for analyzing genes using the DNA microarray of the present invention does not require an expensive laser detection system or a complex optical detection system and detects the surface potential in an equilibrium state by immobilizing the nucleic acid probes on an insulating substrate, which is different from an amperometric detection system, the problems such as corrosion of the substrate, evolution of gas, and unstable signal values due to interference from oxidation-reduction substances are not created, thus allowing excellently stable and highly accurate detection of genes.

The potentiometric DNA microarray according to one aspect of the present invention comprises a plurality of the insulated gate field effect transistors on which single stranded nucleic acid probes or branched nucleic acid probes are immobilized on the surface of the gate insulators directly or via a carrier, and reference electrodes.

The potentiometric DNA microarray according to another aspect of the present invention comprises a substrate, on which a plurality of the insulated gate field effect transistors are formed and each different kind of single stranded nucleic acid probe or branched nucleic acid probe immobilized on the gate insulators over the channel regions of each of the insulated gate field effect transistors directly or via a carrier in the surface of the substrate.

The potentiometric DNA microarray according to still another aspect of the present invention comprises a first insulated gate field effect transistor on which a nucleic acid probe having a base sequence complementary to a portion of a target nucleic acid to be detected is immobilized on the surface of the gate insulator directly or via a carrier, a second insulated gate field effect transistor on which a nucleic acid probe having a base sequence noncomplementary to any portion of the target nucleic acid to be detected is immobilized on the surface of the gate insulator directly or via a carrier, and a circuit to detect and compare outputs of the first insulated gate field effect transistor and the second insulated gate field effect transistor.

The method of analyzing nucleic acids according to one aspect of the present invention comprises the steps of (a) introducing a sample solution containing at least one kind of nucleic acid onto the substrate provided with a plurality of insulated gate field effect transistors on which each different kind of single stranded nucleic acid probe or branched nucleic acid probe is immobilized on the surface of gate insulators directly or via a carrier, and subjecting to hybridization with the single stranded nucleic acid probes or branched nucleic acid probes, (b) introducing a washing solution onto the substrate to remove unreacted nucleic acids from the surface of the substrate, (c) introducing an intercalator solution onto the substrate to react with formed double stranded nucleic acids, (d) introducing the washing solution onto the substrate to remove unreacted intercalator from the surface of the substrate, and (e) introducing a buffer onto the substrate to measure outputs of the insulated gate field effect transistors.

In addition to the above steps of (a) to (e), when the method of analyzing nucleic acids further comprises the steps of (f) dissociating the nucleic acids hybridized with the single stranded nucleic acid probes or branched nucleic acid probes by heating the substrate and (g) introducing the washing solution onto the substrate to remove the nucleic acids and the intercalator dissociated from the single stranded nucleic acid probes or branched nucleic acid probes, and then the steps (a) to (e) are repeated, a plurality of measurements can be performed continuously.

The method of analyzing nucleic acids according to another aspect of the present invention comprises the steps of (a) introducing a sample solution containing nucleic acid fragments labeled with a molecule capable of incorporating charged particles onto a substrate provided with a plurality of insulated gate field effect transistors, on which each different kind of single stranded nucleic acid probe or branched nucleic acid probe is immobilized on the surface of gate insulators directly or via a carrier, to subject to hybridization with the single stranded nucleic acid probes or branched nucleic acid probes, (b) introducing a washing solution onto the substrate to remove unreacted nucleic acid fragments from the surface of the substrate, (c) introducing a solution containing an ion to form a complex with the labeling molecule onto the substrate to react with the molecule labeled on the formed double stranded nucleic acids and form a complex between the ion and the labeling molecule, and (d) measuring outputs of the insulated gate field effect transistors.

The molecule capable of incorporating charged particles may be a molecule that forms a complex selectively with a monovalent or bivalent cation or anion, for example, valinomycin, nonactin, monactin, bis(crown ether), a calixarene derivative, a non-cyclic polyether derivative, a quaternary ammonium salt, a porphyrin or a derivative of these molecules.

In this case, plural kinds of molecules capable of incorporating the charged particles are used to label different nucleic acid fragments, respectively, and the charged particles (ions) corresponding to each of the molecules are introduced onto the substrate, thereby allowing measurements of plural kinds of genes or genetic polymorphism simultaneously or one after another.

In addition to the above steps of (a) to (d), when the method of analyzing nucleic acids according to the present invention further comprises steps of (e) heating the substrate to dissociate the nucleic acid fragments hybridized with the single stranded nucleic acid probes or branched nucleic acid probes and (g) introducing the washing solution onto the substrate to remove the nucleic acid fragments dissociated from the single stranded nucleic acid probes or branched nucleic acid probes, and then the steps (a) to (d) are repeated, multiple measurements can be performed continuously.

The process for producing the potentiometric DNA microarray according to one aspect of the present invention comprises the steps of: forming a silicon film on a first surface of the insulating substrate; dividing the silicon film into a plurality of silicon film formation areas by patterning of the silicon film; forming a plurality of pn junctions working as source and drain regions of the field effect transistors, a heater, and a temperature sensor, respectively, in each of the silicon film formation areas; carrying out wiring for signal from the source and drain regions with the region between the source and drain regions serving as a channel; and immobilizing nucleic acid probes directly or via a carrier at the sites corresponding to the channels of the field effect transistors on a second surface opposite to the surface where the silicon film is formed on the insulating substrate.

The process for producing the potentiometric DNA microarray according to another aspect of the present invention comprises the steps of: forming a silicon film on a surface of the insulating substrate; dividing the silicon film into a plurality of silicon film formation areas by patterning of the silicon film; forming a plurality of pn junctions working as the source and drain regions of the field effect transistors, a heater, and a temperature sensor, respectively, in each of the silicon film formation areas; carrying out wiring for signal from the source and drain regions with the region between the source and drain regions serving as a channel; forming an insulating film on the surface where the wiring for signal is carried out; and immobilizing nucleic acid probes directly or via a carrier at the sites corresponding to the channels of the field effect transistors on the surface of the insulating film.

### BRIEF DESCRIPTION OF DRAWINGS

Fig. 1 is a cross sectional schematic drawing to explain an example of a field effect transistor for gene detection according to the present invention;
Fig. 2 is a construction example to show a measurement system with the field effect transistor for detection of gene according to the present invention;
Fig. 3 is an explanatory drawing of a measurement system of a DNA microarray of the present invention in combination with an intercalator;
Fig. 4 is an explanatory drawing of a measurement system of the DNA microarray of the present invention in combination with a labeling molecule;
Fig. 5 is a drawing to show an example of the process for producing a thin film gate type FET chip (DNA microarray) for detection of gene of the present invention;
Fig. 6 is a plan view to show an example of the DNA microarray of the present invention;
Fig. 7 is an explanatory drawing of an arrangement example of FET, heater, and temperature sensor;
Fig. 8 is a perspective view from the backside of the DNA array of the present invention;
Fig. 9 is a plan view to show the DNA microarray provided with a radiation fin of the present invention;
Fig. 10 is a perspective view from the backside of the DNA microarray provided with the radiation fin of the present invention;
Fig. 11 is a cross sectional view showing an example of the thin film gate type FET chip for detection of gene of the present invention;
Fig. 12 is a cross sectional view showing another example of the thin film gate type FET chip for detection of gene of the present invention;
Fig. 13 is an explanatory drawing of an example of the measurement system with the thin film gate type FET chip for detection of gene of the present invention;
Fig. 14 is an explanatory drawing of a flow cell provided with the FET chip for detection of gene of the present invention;
Fig. 15 is an exploded view of the flow cell shown in Fig. 14; and
Fig. 16 is an explanatory drawing of a protocol for the measurement by the FET for detection of gene of the present invention.

### BEST MODE FOR CARRYING OUT THE INVENTION

The present invention is explained below in detail with reference to the accompanying drawings. The same functional portions are designated by the same reference numerals in the following illustrations.

### Example 1

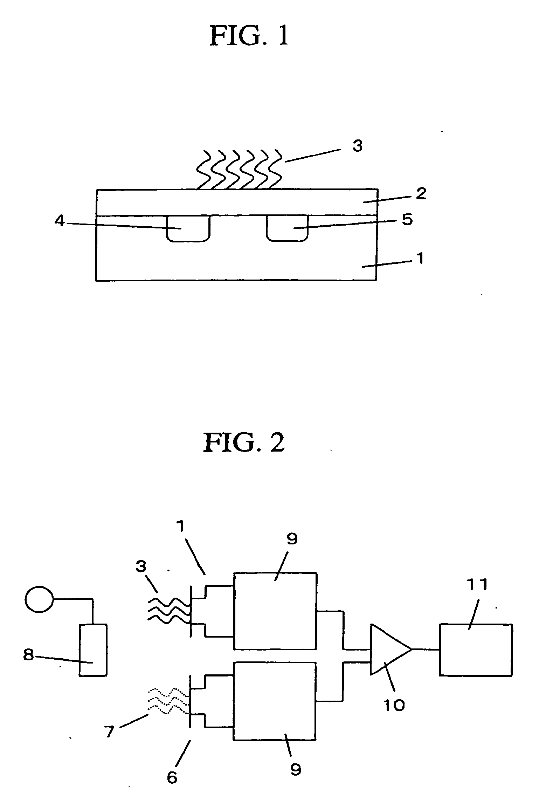

Fig. 1 is a cross sectional schematic drawing to explain an example of an electric field effect transistor (FET) for gene detection according to the present invention.

It is configured such that a nucleic acid probe 3 is immobilized on the surface of a gate insulator 2 of an insulated gate FET 1. For the nucleic acid probe is used an oligonucleotide, cDNA, or DNA fragment branched in the middle, each generally composed of 300 nucleotides or less and capable of hyblidizing with a target gene to be measured under appropriate conditions. In the case of an oligonucleotide, it is preferably a nucleic acid fragment with a length of 80 bases or less. For the gate insulator, a material such as silicon dioxide (SiO₂), silicon nitride (SiN), aluminum oxide (Al₂O₃), or tantalum oxide (Ta₂O₅) is used alone or in combination, and generally a bilayer structure in which silicon nitride (SiN), aluminum oxide (Al₂O₃), and tantalum oxide (Ta₂O₅) are layered on top of silicon dioxide (SiO₂) is employed to keep performance of a transistor better.

In order to immobilize the nucleic acid probe on the surface of the insulator, one of the terminal ends of the nucleic acid probe is chemically modified to contain an amino group (NH₂ group), a thiol group (SH group), biotin, or the like. When a nucleic acid probe chemically modified to contain an amino group is used, the surface of the insulator is modified with a chemical such as aminopropylethoxysilane or polylysine to introduce an amino group onto the surface of the insulating film, and then the latter group is reacted with glutaraldehyde or phenylene diisocyanate (PDC) to immobilize the nucleic acid probe chemically modified to contain the amino group on the surface of the insulator. When the nucleic acid probe chemically modified to contain the thiol group is immobilized on the surface of the insulator, a gold thin film is formed on the insulator and the nucleic acid probe is immobilized by taking advantage of an affinity between thiol group and gold. Furthermore, when the nucleic acid probe chemically modified to contain biotin is immobilized, streptoavidin is introduced onto the surface of the insulator and the nucleic acid probe is immobilized by taking advantage of an affinity between biotin and streptoavidin on the surface of the gate insulator. At the time of a practical immobilization, a solution containing the nucleic acid probe is dropped or spotted only on the surface of the gate insulator on FET channel, thereby immobilizing the nucleic acid probe only on the gate insulator of the channel portion.

On the other hand, by forming a fixed carrier on the surface of the gate insulator of FET, the nucleic acid probe may be immobilized on the surface or in the inside of the fixed carrier in an indirect way. The materials that can be used for the fixed carrier include agarose, polyacrylamide, polyhydroxyethyl methacrylate (pHEMA), and the like. The fixed carrier may be chemically modified to contain an amino group or streptoavidin, and as described above, the nucleic acid probe may be immobilized by using glutaraldehyde or PDC, or by taking advantage of an affinity with biotin, respectively, on the fixed carrier. In this way, the nucleic acid probe may also be formed indirectly on the gate insulator of FET via the fixed carrier.

When a number of genes containing a target gene to be measured are present in a sample and when a nucleic acid probe having a base sequence complementary to the target gene is immobilized on the gate insulator of FET for gene detection, the target gene and the nucleic acid probe hybridize with each other under appropriate reaction conditions to form a complex of the target gene and the nucleic acid probe. Under an appropriate condition for pH of a buffer solution used for the reaction, nucleic acids are charged negative. Accordingly, the formation of the complex by hybridization induces a change in electric charge density in the vicinity of the gate insulator of FET, thereby changing the surface potential of the gate insulator. This change behaves equally as a gate voltage change of FET, resulting in a change of conductivity of the channel. Therefore, the formation of the complex, that is, the presence of the target gene, can be detected as a change in drain current passing between a source 4 and a drain 5.

### Example 2

Fig. 2 is a construction example to show a genetic testing system with the field effect transistor for detection of gene (Genetic FET) shown in Fig. 1.

This genetic testing system is provided with a reference FET 6 in addition to the Genetic FET 1 shown in Fig. 1, and a differential measurement between the Genetic FET and the reference FET is carried out. The nucleic acid probe 3 having the base sequence complementary to the target gene in a sample is immobilized on the surface of the gate insulator of the Genetic FET 1. On the other hand, a nucleic acid probe 7 having a base sequence that is different from the base sequence complementary to the target gene is immobilized on the surface of the gate insulator of the reference FET 6.

Such a differential measurement allows to accurately detect only the output change caused by the hybridization of the target gene and the nucleic acid probe by compensating for an output change occurring from changes in ambient temperature and light due to the difference in electric properties of FET or by offsetting an output change arising from non-specific adsorption of charged particles in the sample on the gate insulator. Since the Genetic FET and the reference FET are desired to be uniform in their electric properties, it is desirable to use a pair of FET integrated on the same substrate.

In order to stably measure the surface potentials of the Genetic FET and the reference FET, a reference electrode 8 that serves as a reference for the potential measurement is provided. The reference electrode conventionally used is the one that a silver-silver chloride electrode or a calomel electrode is dipped into an internal solution for the electrode having a specified composition and concentration, and the electrode is constructed such that the internal solution and a sample solution make contact with each other via a liquid junction formed with salt bridge or porous material. The surface potentials of the Genetic FET 1 and the reference FET 6 are measured with their respective drive circuits 9, and both of the outputs are input to a signal processing circuit 11 via the differential measurement circuit 10. When a plurality of Genetic FETs are integrated to measure a plurality of genes at the same time, the reference FET may be used in common to perform the differential measurements between different Genetic FETs and the common reference FET.

### Example 3

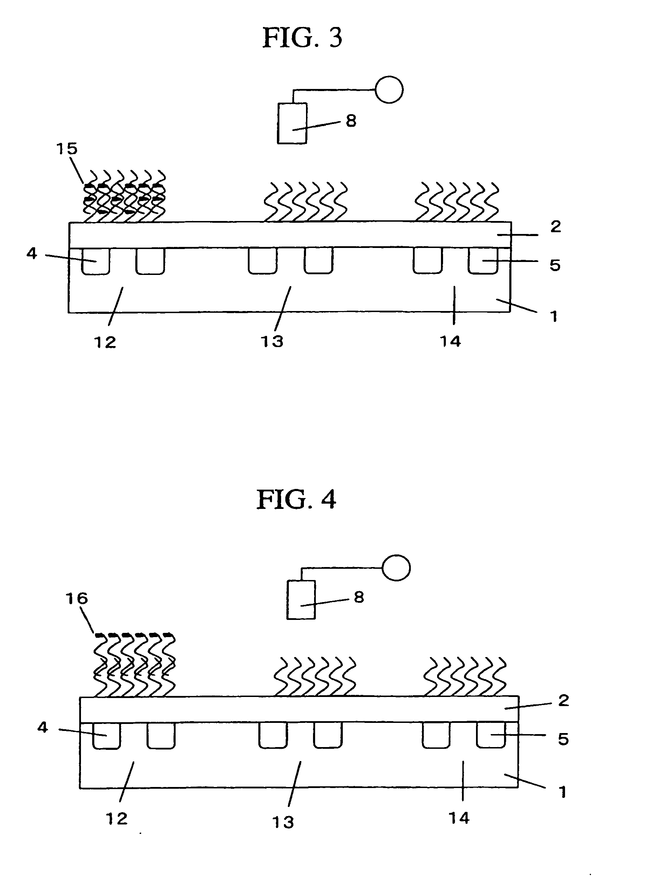

Fig. 3 is an explanatory drawing of a measurement system of the DNA microarray of the present invention in combination with an intercalator. In the illustrated example of the DNA microarray, three Genetic FETs 12 to 14 are integrated. The first FET 12 is used as a Genetic FET to detect a first target gene, the second FET 13 is used as a Genetic FET to detect a second target gene, and the third FET 14 is used as the reference FET. Nucleic acid probes having base sequences complementary to the first and second genes are immobilized on the gate insulators of the first and second FETs, respectively. A nucleic acid probe having a base sequence that is different from the base sequences complementary to the first and second genes is immobilized on the surface of the gate insulator of the reference FET.

Fig. 3 depicts a state in which a sample solution containing only the first gene is introduced to the integrated DNA microarray and hybridized with the target gene, followed by the reaction with an intercalator. The first gene hybridizes only with the nucleic acid probe on the first FET 12 to form double strand. The intercalator 15 reacts only with a double-stranded nucleic acid and does not reacts with single-stranded nucleic acids. Since the intercalator 15 is electrically charged, only the surface electric charge density of the first Genetic FET 12 changes to give rise to a change in the output signal of FET. Since the surface electric charge densities of the second Genetic FET 13 and the reference FET 14 do not change, their output signals do not change. Therefore, the differential measurement between the first Genetic FET 12 and the reference FET 14 and that between the second Genetic FET 13 and the reference FET 14 result in a change of only the output signal for the former, thereby detecting the first target gene. Alternatively, the measurement of the output ratio between the first Genetic FET 12 and the reference FET 14 and that between the second Genetic FET 13 and the reference FET 14 result in detecting the first target gene owing to a change of the output ratio for the former. Thus, comparison of the outputs of a plurality of FETs makes it possible to detect target genes. The intercalator that can be used includes ethidium bromide, Hoechst 33258, and the like.

A specific example is explained below. It is known that there exist single nucleotide polymorphisms (SNPs) in alcohol dehydrogenase-related gene. A first and a second nucleic acid probes each having a length of 17 bases in which the base at a SNP position is interposed between 8 common bases were synthesized. The base sequences of these probes are shown below.
First nucleic acid probe: 5'-CATACACTAAAGTGAAA-3'
Second nucleic acid probe: 5'-CATACACTGAAGTGAAA-3'

The ninth position from the 5' terminus is the site of the SNP. In the first nucleic acid probe, the base is A at the SNP site, while in the second nucleic acid probe, the base is G at the site. The first and the second nucleic acid probes are immobilized on the first and the second gates of FETs, respectively. A nucleic acid probe that has a base sequence different from the first and the second nucleic acid probes, for example, a nucleic acid probe of 17 bases composed exclusively of A, is immobilized on the gate of the reference FET. Alternatively, a nucleic acid probe may not be immobilized on the gate of the reference FET. The 5' termini of the above nucleic acid probes are modified to contain amino groups, respectively. On the other hand, the gate insulating film of FET in the present example is made of silicon nitride, and the surface of the silicone nitride is chemically modified using γ-aminopropyltriethoxysilane to introduce an amino group thereon. The amino group of the nucleic acid probe and that of the silicone nitride are reacted with a bifunctional reagent such as glutaraldehyde to immobilize the nucleic acid probe on the surface of silicone nitride through the formation of a Schiff base bond.

The human genome was extracted from white blood cells in the blood, and a region of 100 base length that contains the SNP site was amplified to serve as a sample to be tested. The sample was introduced to the first and the second Genetic FETs and the reference FET, followed by hybridization for 8 hours at 45 degrees C. After the hybridization, these FETs were washed with a buffer to remove unreacted sample, and then ethidium bromide was introduced as an intercalator to them. The measurement was first conducted by introducing the buffer to the first and the second Genetic FETs and the reference FET, and the output voltage of each of these FETs, the differential output between the first Genetic FET and the reference FET and that between the second Genetic FET and the reference FET were measured. After these measurements, the sample was introduced, hybridized, and washed, followed by introduction of the intercalator, and then the output voltage of each of these FETs, the differential output between the first Genetic FET and the reference FET, and that between the second Genetic FET and the reference FET were measured. The changes in the output voltage before and after the introduction of the sample and the intercalator were measured.

In a sample having the base sequence corresponding to the first nucleic acid probe (Normal), the differential output between the first Genetic FET and the reference FET was 5.0 mV. On the other hand, the differential output between the second Genetic FET and the reference FET was 0.5 mV, showing a significant difference from the above. It is desirable that a series of these measurements are performed at the same time, while successive measurements are also allowed when performed within the same test time period. In another sample having the base sequence corresponding to the second nucleic acid probe (Mutant), the differential output between the first Genetic FET and the reference FET was 0.3 mV. On the other hand, the differential output between the second Genetic FET and the reference FET was 4.0 mV, showing again a significant difference from the above. In still another sample having the base sequences corresponding to the first and the second nucleic acid probes half-and-half (Hetero), the differential output between the first Genetic FET and the reference FET was 2.3 mV, while the differential output between the second Genetic FET and the reference FET was 2.0 mV, showing approximately one to one ratio between Normal and Mutant. From the foregoing, the SNP analysis according to the present invention allowed to discriminate three kinds of samples of Normal/Normal homo-type, Mutant/Mutant homo-type, and Normal/Mutant hetero-type. When an intercalator is used, it is unnecessary to label the DNA sample by chemically linking a labeling compound.

When the detection was carried out only by the charge inherent in the target DNA alone without using the intercalator as shown in Fig. 2, the differential output between the Genetic FET and the reference FET was 2.2 mV in the measurement of the Normal/Normal or Mutant/Mutant homo-type sample. Thus, the use of the intercalator enhanced the sensitivity about two fold.

### Example 4

Fig. 4 is an explanatory drawing of a measurement system of the DNA microarray of the present invention in combination with a labeling molecule. In the illustrated example of the DNA microarray, three Genetic FETs 12 to 14 are integrated. The first FET 12 is used as a Genetic FET to detect a first target gene, the second FET 13 is used as a Genetic FET to detect a second target gene, and the third FET 14 is used as the reference FET. Nucleic acid probes having base sequences complementary to the first and the second genes are immobilized on the gate insulators of the first and the second FETs, respectively. A nucleic acid probe having a base sequence that is different from the base sequences complementary to the first and the second genes is immobilized on the surface of the gate insulator of the reference FET.

Fig. 4 depicts a state in which a sample solution containing only the first gene is introduced to the integrated DNA microarray and hybridized with the target gene. The first gene hybridizes only with the nucleic acid probe on the first FET to form double strand. The first gene is labeled with a molecule 16 that makes a complex with a charged molecule or an ion, and the electric charge density on the surface of the gate insulator changes owing to the specific hybridization. Since the surface electric charge densities of the second Genetic FET and the reference FET do not change, their r output signals do not change. Therefore, the differential measurement between the first Genetic FET and the reference FET and that between the second Genetic FET and the reference FET result in detecting the first target gene owing to a change of only the output signal for the former.

The molecule (ligand) that is used to form a complex with the ion includes valinomycin, nonactin, monactin, bis(crown ether), calixarene derivatives, non-cyclic polyether derivatives, quaternary ammonium salts, and the like. For example, when the target gene is labeled with Bis(12-crown-4), a property of Bis( 12-crown-4) to form a complex selectively with sodium ion may be utilized. That is, the labeled target gene is hybridized with the nucleic acid probe on the gate insulator, and then, a buffer containing sodium ion is introduced onto the DNA microarray to allow a selective complex formation between Bis(12-crown-4) and sodium ion, which causes a local change in the electric charge density on the surface of the gate insulator. This change is detected by the FET.

The present example was specifically applied to an SNP analysis, which is explained below. Using the alcohol dehydrogenase-related gene shown in Example 3, two nucleic acid probes shown below were immobilized on the gate insulators of the first and second Genetic FETs, respectively.
First nucleic acid probe (Normal): 5'-CATACACTAAAGTGAAA-3'
Second nucleic acid probe (Mutant): 5'-CATACACTGAAGTGAAA-3'
A nucleic acid probe that has a sequence different from those of the first and the second nucleic acid probes, for example, a nucleic acid probe of 17 base length composed exclusively of A, is immobilized on the gate of the reference FET. A DNA sample was extracted from white blood cells in the blood, and a region of 100 base length that contains the SNP site was amplified, and then Normal DNA and Mutant DNA were labeled with bis(crown ether) and valinomycin, respectively.

The sample from Normal DNA was introduced to the first and second Genetic FETs and the reference FET, followed by hybridization for 8 hours at 45 degrees C. After the hybridization, these FETs were washed with a buffer to remove unreacted sample, and an aqueous solution of 50 mM NaCl was introduced thereto, followed by the measurements of the output voltage of each of the first and the second Genetic FETs and the reference FET, the differential output between the first Genetic FET and the reference FET and that between the second Genetic FET and the reference FET. The differential output between the first Genetic FET and the reference FET was 4.0 mV, while the differential output between the second Genetic FET and the reference FET was 0.2 mV. After these measurements, an aqueous solution of 50 mM KCl was introduced, and the measurements of the outputs were carried out similarly, resulting in that the differential output between the first Genetic FET and the reference FET was 0.1 mV and that the differential output between the second Genetic FET and the reference FET was 0.3 mV. Thus, it was confirmed that Normal DNA in the sample hybridized only with the first nucleic acid probe, showing a selective response of the first Genetic FET.

On the other hand, a sample of Mutant DNA was measured in a way similar to the above. When a solution of 50 mM NaCl was introduced, the differential output between the first Genetic FET and the reference FET was 0.1 mV, while the differential output between the second Genetic FET and the reference FET was 0.2 mV. After an aqueous solution of 50 mM KCl was introduced, the differential output between the first Genetic FET and the reference FET was 0.1 mV, while the differential output between the second Genetic FET and the reference FET was 5.0 mV, confirming that Mutant DNA in the sample hybridized only with the second nucleic acid probe showing a selective response of the second Genetic FET. In another sample containing Normal and Mutant DNAs half-and-half (Hetero), when an aqueous solution of 50 mM NaCl was introduced, the differential output between the first Genetic FET and the reference FET was 2.3 mV, while the differential output between the second Genetic FET and the reference FET was 0.1 mV. After an aqueous solution of 50 mM KCl was introduced, the differential output between the first Genetic FET and the reference FET was 0.1 mV, while the differential output between the second Genetic FET and the reference FET was 2.5 mV, showing approximately one to one ratio between Normal and Mutant.

From the foregoing, the SNP analysis according to the present invention allowed to discriminate three kinds of samples of Normal/Normal homo-type, Mutant/Mutant homo-type, and Normal/Mutant hetero-type. In the present example, the processes of the SNP analysis involve two selective processes of chemical reactions that are hybridization and ion-ligand complex formation. Therefore, the SNP analysis can be performed with high accuracy.

Besides the SNP analysis, it is also possible to perform an expression analysis by immobilizing many kinds of nucleic acid probes on the gates of FETs, and labeling a target sample and its reference sample with valinomycin and bis(crown ether), respectively.

### Example 5

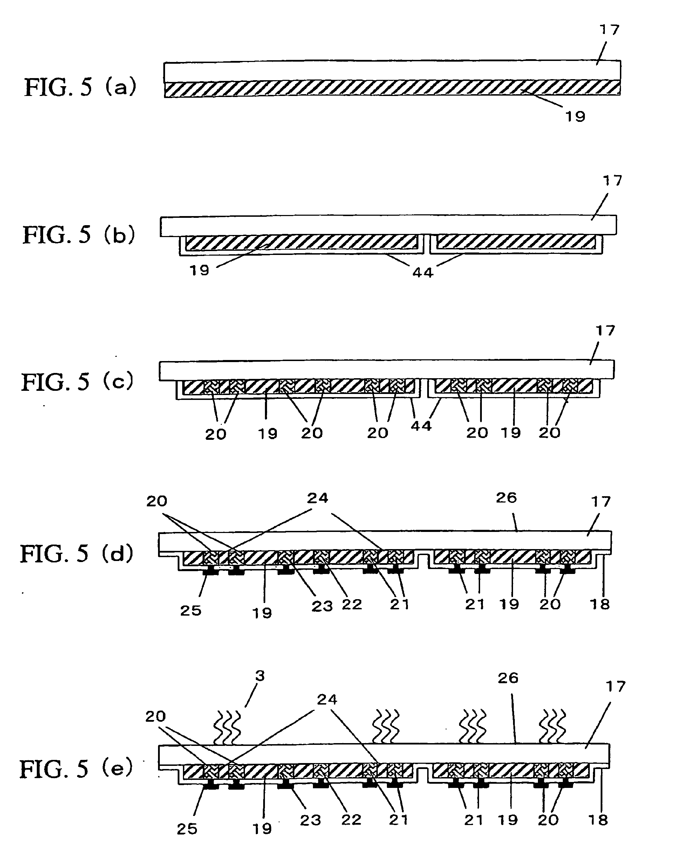

Fig. 5 is a drawing to show an example of the process for producing the DNA microarray provided with a thin film gate type field effect transistor for detection of gene of the present invention. First, a p-type polysilicon thin film 19 is formed on a glass substrate 17 as shown in Fig. 5(a) by the low pressure chemical vapor deposition method. The thickness of the polysilicon thin film is desirably in the range of from 10 to 10,000 nm, and was 1,000 nm in the present example. Next, patterning and oxidation of the polysilicon film are performed as shown in Fig. 5(b). The patterning is performed by the photolithographic process including resist application, photoirradiation through a photomask, and resist development, followed by dry etching or wet etching with a mixed solution of hydrofluoric acid and nitric acid to form silicon film formation areas 19. Then, the silicon film is thermally oxidized at 900 degrees C in an atmosphere of oxygen to form a silicon dioxide film 44 on the surface of the polysilicon thin film 19.

Next, etching of oxidized film and formation of doping regions are carried out as shown in Fig. 5(c). The silicon dioxide film on the doping regions is removed by the photolithographic process including resist application, photoirradiation through a photomask, and resist development, and the subsequent dry etching or wet etching with a mixed solution of hydrofluoric acid and ammonium fluoride. By As⁺ or P⁺ ion implantation, n-type regions are formed in the area of the p-type polysilicon thin film 19 to provide pn junction 20. Then, the silicon film in the doping regions is thermally oxidized at 900 degrees C in an atmosphere of oxygen to form a silicon dioxide film 44 on the surface thereof.

Next, metal wiring for electrodes is carried out as shown in Fig. 5(d). The silicon dioxide film is removed to provide electrode contact holes by the photolithographic process including resist application, photoirradiation through a photomask, and resist development, and the subsequent dry etching or wet etching with a mixture of hydrofluoric acid and ammonium fluoride. An aluminum thin film is formed by vacuum deposition. According to the photolithographic process including resist application, photoirradiation through a photomask, and resist development, and the subsequent wet etching with phosphoric acid, aluminum wiring 25 is formed by patterning to achieve contact with the external circuit. After annealing in an atmosphere of hydrogen, source and drain regions 21 of the FETs, a heater 22 and a temperature sensor 23 are provided. The region between the source and drain regions serves as the channel 24 of FET.

Finally, DNA probes are immobilized as shown in Fig. 5(e). The nucleic acid probes 3 are immobilized at the sites corresponding to the channels of FET on the second surface 26 (the surface opposite to the surface where the silicon film is formed) of the glass substrate 17 to provide Genetic FETs. In order to achieve high accuracy of measurement, it is desirable to integrate at least Genetic FETs, the reference FET, the heater, and the temperature sensor into the same silicon film formation area 19.

On the surface of the insulating glass surface 17, a solution is introduced onto the surface where the nucleic acid probes 3 are formed. Therefore, the solution may sometimes contact with wiring and signal wires for an electronic device such as transistor to develop a short-circuit, resulting in a faulty operation. In the present invention, the wiring 25 is formed on the surface opposite to the surface with which the solution makes contact and on which the nucleic acid probes 3 are formed, and signal wires are designed to be connected to the opposite surface. Accordingly, the problem of the faulty operation caused by contacting with the solution is eliminated, and a measurement system with high reliability can be provided.

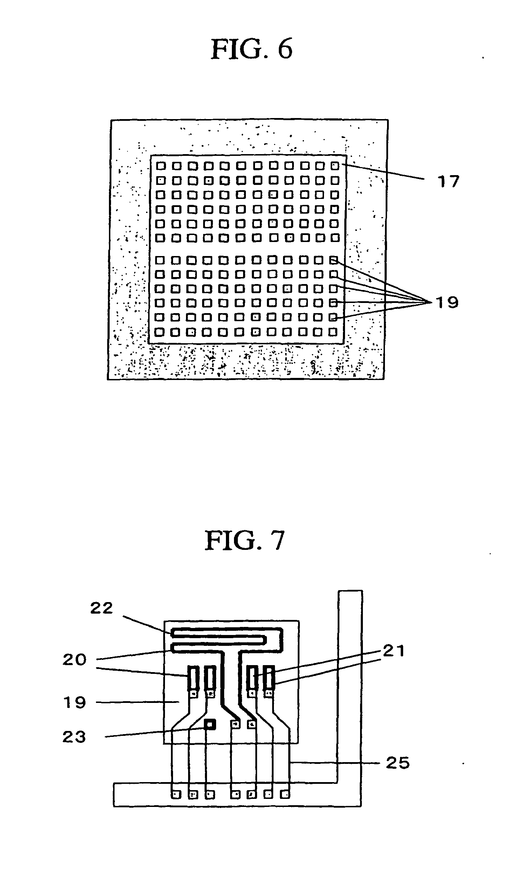

Fig. 6 is a plan view to show an example of the DNA microarray produced in this way. In the microarray of this example, 144 pieces of the silicon film formation area 19 are separately formed on the insulating substrate 17. One piece of the silicon film formation area 19 has a size of 500 µm square. An enlarged view from the backside of the silicon film formation area 19 is shown in Fig. 7. A plurality of pn junctions 20 were formed in each of the silicon film formation areas 19. When the silicon film is p-type, n-type doping regions are formed, and when the silicon film is n-type, p-type doping regions are formed. These doping regions served for the source and drain regions 21 of FETs, the heater 22, or the temperature sensor 23. Each doping region is bonded to external drive circuit via the electric wiring 25.

In the present example, two FETs are formed in one piece of the silicon film formation area 19 as shown in Fig. 7, where one FET served as the Genetic FET and the other FET served as the reference FET. A control of the heater 22 with an output of the temperature sensor 23 allows adjustment of the temperature of each of the silicon film formation areas 19 to a desirable value. Accordingly, the nucleic acid probes with comparable melting temperatures (Tms) are immobilized on Genetic FETs in the same silicon film formation area 19, while the nucleic acid probes with different Tms are immobilized in different silicon film formation areas 19, thereby realizing gene detection with high accuracy. Although changes in temperature change FET characteristics as well, the differential measurement between the Genetic FET and the reference FET allows compensating for changes in the output of each FET due to changes in temperature.

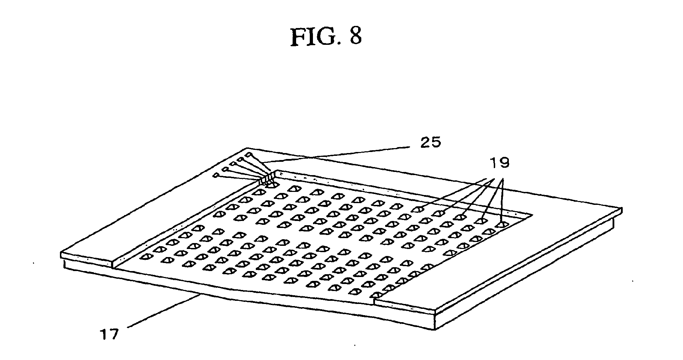

A perspective view from the backside of the DNA array depicted in Fig. 6 is shown in Fig. 8. The electric wire bonding 25 is formed on the backside, while the nucleic acid probes are immobilized on the opposite side.

### Example 6

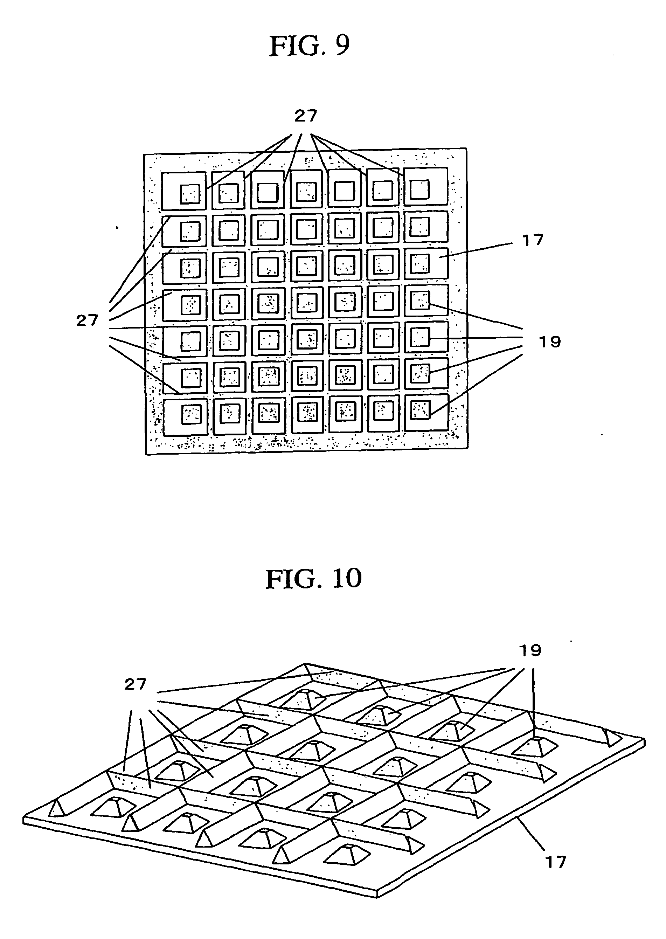

Another example of the present invention is explained using Figs. 9 and 10. Fig. 9 is a plan view of the DNA microarray provided with a radiation fin of the present invention, and Fig. 10 is a perspective view from the backside of the DNA microarray.

In the same way as in Fig. 5, a silicon film was formed on a first surface 18 of an insulating substrate 17, and unnecessary area of the silicon film was removed by photolithography and etching, giving a plurality of separate silicon film formation areas 19. The thickness of the silicon film is preferably in the range of from 0.1 to 10 µm, and was one µm in the present example. As in Example 5, the Genetic FET, the reference FET, a heater, and a temperature sensor are formed in each of the silicon film formation areas 19.

In the present example, the radiation fins 27 of grid pattern are formed of silicon film so as to surround each of the silicon film formation areas 19, the purpose of which is to carry out hybridization and washing at the optimal temperature for each reaction according to Tms of the nucleic acid probes formed on each Genetic FET, thereby improving accuracy of temperature control for each of the silicon film formation areas 19. The radiation fin 27 has good thermal conductivity because of being made of silicon film and can efficiently radiate heat generated in the adjacent silicon film formation areas 19, reduce effects on the adjacent silicon film formation areas, and control the temperature of each of the silicon film formation areas 19 independently. In the present example, the size of one piece of the silicon film formation area 19 is 1 mm square, the distance between the silicon film formation area 19 and the radiation fin 27 is 0.5 mm, and the width of the radiation fin is 0.5 mm. By employing this structure, the temperature of each of the silicon film formation areas 19 could be controlled from room temperature to 95 degrees C with a precision of one degree C.

Fig. 10 is a perspective view from the backside of the above chip. Since the silicon film formation areas and the radiation fins were formed by wet etching technology, the cross sections are triangular and trapezoidal. Rectangular cross sectional shapes for the silicon film formation area and the radiation fins are fabricated by using, for example, the reactive ion etching technology.

### Example 7

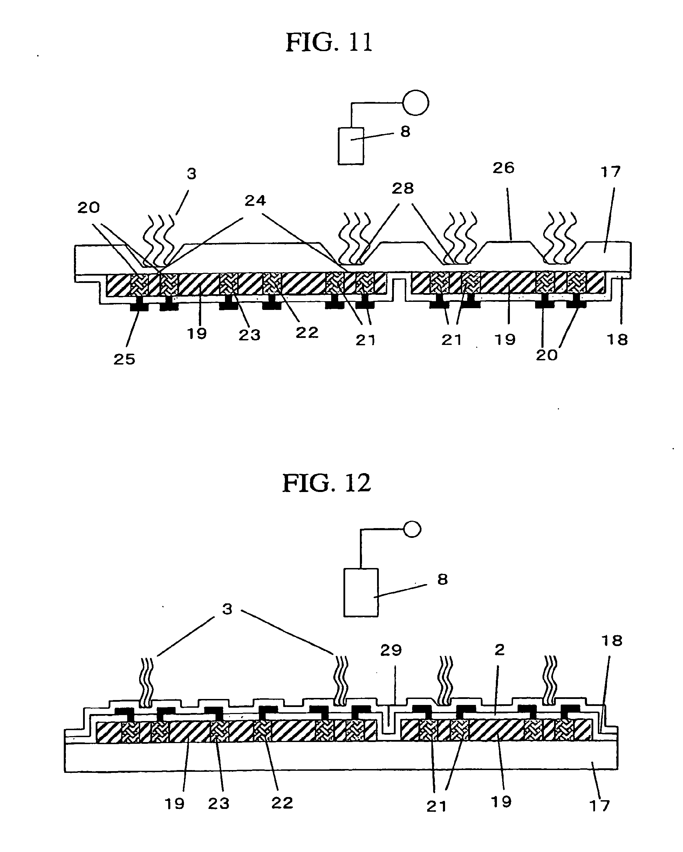

Fig. 11 is a cross sectional view to show an example of thin film gate type Genetic FET chip of the present invention.

The thin film gate type Genetic FET chip of the present example has a structure in which the glass substrate of the gate regions for the Genetic FET and the reference FET in the DNA microarray explained in Example 5 is made thinner. The thickness of the glass substrate in the thin film gate regions 28 is preferably in the range of from 0.01 to 1 µm and was 0.1 µm in the present example. This structure can make the transconductance of the FET larger and detect a change in electric charge occurring on the gate with high sensitivity. In addition to making only the gate region of the FET a thinner film, it is also possible to make the whole glass substrate of the silicon film formation area a thinner film, thereby allowing the recessed area formed by being made thinner to be used as a reaction cell.

### Example 8

Fig. 12 is a cross sectional view to show another example of the thin film gate type Genetic FET chip of the present invention.

Examples 5, 6, and 7 are structured such that the nucleic acid probes 3 are formed on the glass surface opposite to the silicon film formation area 19, whereas the nucleic acid probes 3 in the present example were immobilized on a second insulating film 29 formed on an oxidized silicon film, i.e. an insulating film 2 formed on the silicon film formation areas 19. The material usable for the second insulating film 29 includes silicon nitride, aluminum oxide, and tantalum oxide. This example enables precise control of the thickness of the gate insulating film of FET and detection of a change in electric charge occurring on the gate with high sensitivity by making the transconductance of the FET larger.

### Example 9

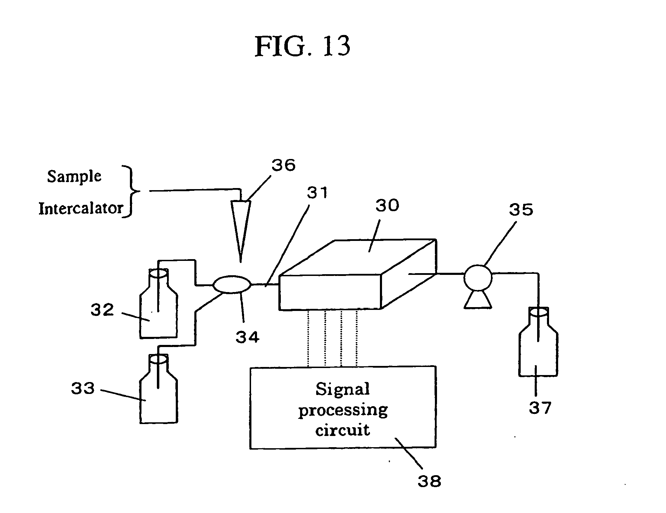

Fig. 13 is an explanatory drawing of an example of the measurement system with the thin film gate type FET chip for detection of gene of the present invention.

The DNA microarray chip having at least the Genetic FET and the reference FET is mounted on a flow cell 30, which is connected to a flow channel 31. A hybridization solution 32 and a washing solution 33 flow into the flow channel 31 via a valve 34. These solutions can be introduced into the flow cell 30 by driving a pump 35. A sample and an intercalator are dispensed into the valve 34 by a dispenser 36, and then introduced into the flow cell 30 to react with the Genetic FET and the reference FET. After the reaction, spent solution is sent to a liquid waste bottle 37 by the pump 35. The outputs of the Genetic FET and the reference FET after the reaction are processed and computed by a signal processing circuit 38.

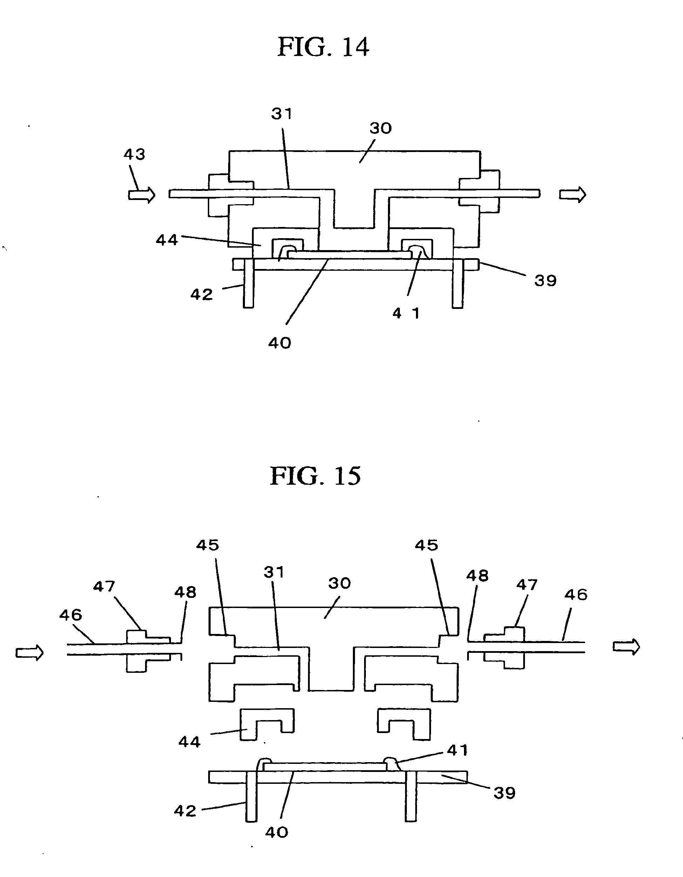

The structure of the flow cell 30 is shown in Fig. 14. A Genetic FET chip 40 is mounted on a printed circuit board 39 in the flow cell 30 and electrically connected to the printed circuit board 39 with wires 41. Pins 42 are provided on the printed circuit board 39 and connected to the signal processing circuit 38. A sample solution 43 is introduced to the Genetic FET chip 40 through the flow channel 31. In order to prevent the sample solution 43 from contacting with the wires 41 that serve as signal wires, the wire portion is protected by protective caps 44. The material suitable for the protective cap 44 includes acryl, polypropylene, polycarbonate, and the like.

Fig. 15 is an exploded view to show the flow cell 30. The main body of the flow cell 30 is provided with a hole of one mm diameter serving as the flow channel 31, through which a sample and a reagent are introduced onto the surface of the Genetic FET chip (DNA microarray) 40. The inlet and the outlet portions of the flow channel 31 are provided with female threads 45, into which bolts 47 having inserted tubes 46 are screwed to connect to the external flow path. The surface of the end portion of the tube 46 is treated so as to become flattened, and when the bolt is screwed in, the flattened portion works as a seal 48 to prevent liquid leakage.

The measurement system of Genetic FET of the present construction employs a flow system for the measurement, and therefore, a number of samples can be handled continuously and automatically, which is advantageous for a high-throughput measurement. When the intercalator described in Example 3 is used, the measurement is conducted by the following steps:
(1) Introduction of a washing solution into the flow cell.
(2) Introduction of a hybridization solution into the flow cell (replacement of the washing solution).
(3) Setting of the temperature of each of the silicon film formation areas to the optimal temperature for each nucleic acid probe.
(4) Measurement of the outputs of the Genetic FET and the reference FET and computation of the difference.
(5) Dispensing of a sample to the valve and subsequent introduction to the flow cell with the hybridization solution.
(6) Hybridization in the flow cell.
(7) Introduction of a buffer to the flow cell to remove unreacted sample.
(8) Introduction of an intercalator solution into the flow cell and reaction.
(9) Introduction of the buffer to remove unreacted intercalator solution.
(10) Measurement of the outputs of the Genetic FET and the reference FET, and computation of the difference.
(11) Setting of the temperature of each of the silicon film formation areas to 95 degrees C.
(12) Introduction of the washing solution to wash the inside of the flow cell.
(13) Return to (1).

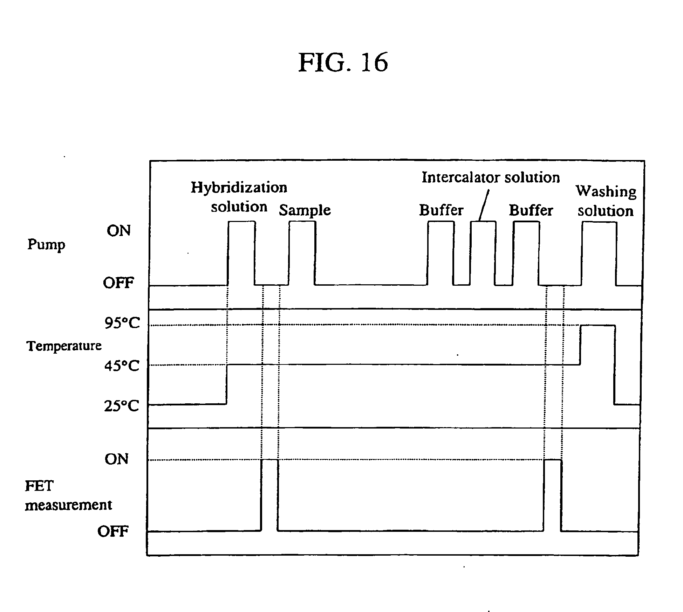

The above sequence for the measurement is shown in Fig. 16. The sample and the intercalator were introduced and washed, followed by measurement of the output voltage of FETs. Then, the hybridized double stranded DNAs were dissociated into single stranded DNAs by raising the temperature of the silicon film formation areas to 95 degrees C and the dissociated DNA is removed together with the intercalator inserted into the double stranded DNA by the washing solution. In this way, only the nucleic acid probe is left on the gate of FET, which allows to return to the original state and to be ready for the next measurement.

When SNP is analyzed using the molecules (ligands) to form complexes with ions as described in Example 4, the measurement is conducted by the following steps:
(1) Introduction of a washing solution into the flow cell.
(2) Introduction of a hybridization solution into the flow cell (replacement of the washing solution).
(3) Setting of the temperature of each of the silicon film formation areas to the optimal temperature for each nucleic acid probe.
(4) Measurement of the outputs of the Genetic FET and the reference FET and computation of the difference.
(5) Dispensing of a sample to the valve and subsequent introduction to the flow cell with the hybridization solution.
(6) Hybridization in the flow cell.
(7) Introduction of a buffer to the flow cell to remove unreacted sample.
(8) Introduction of a solution of 50 mM NaCl into the flow cell and reaction.
(9) Measurement of the outputs of the Genetic FET and the reference FET and computation of the difference.
(10) Introduction of a solution of 50 mM KCl into the flow cell and reaction.
(11) Measurement of the outputs of the Genetic FET and the reference FET and computation of the difference.
   Judgment of Normal/Normal, Mutant/Mutant, and Normal/Mutant from the information on the nucleic acid probe for each FET and the differential outputs in NaCl and KCI.
(12) Introduction of the buffer to remove the KCl solution.
(13) Setting of the temperature of each of the silicon film formation areas to 95 degrees C.
(14) Introduction of the washing solution to wash the inside of the flow cell.
(15) Return to (1).

### INDUSTRIAL APPLICABILITY

The present invention provides a DNA microarray system in which nucleic acid probes are immobilized on the surface of the gate insulator of FET and then hybridized with a target gene on the surface of the gate insulator of FET, and a change in the surface electric charge density is detected by using the field effect. The DNA microarray that allows a potentiometric detection of a change in the surface electric potential with a high signal to noise ratio can be realized by introducing an intercalator or labeling nucleic acids with molecules to form complexes with charged particles such as ions in order to amplify the change in the surface electric charge density in addition to the charge inherent in the nucleic acids. The DNA microarray of the present invention does not require an expensive laser detection system or a complex optical detection system, and detects the surface potential in an equilibrium state by immobilizing the nucleic acid probes on an insulating substrate, which is different from an amperometric detection system. Therefore, the problems such as corrosion of the substrate, evolution of gas, and unstable signal values due to interference from oxidation-reduction substances do not arise, thus allowing excellently stable and highly accurate detection of genes.

## Claims

1. A potentiometric DNA microarray comprising:
a substantially planar insulator (17, 29),
a silicon film (19) formed on a first surface of the insulator,
an insulated gate field effect transistor (1, 6, 12, 13, 14) formed in the silicon film, and
a reference electrode (8),
wherein a single stranded or branched nucleic acid probe (3, 7) is immobilised directly or via a carrier on a second surface of the insulator (17, 29) opposite to the first surface.

2. A potentiometric DNA microarray comprising:
a substantially planar insulator (17, 29),
a silicon film (19) formed on a first surface of the insulator, and
a plurality of insulated gate field effect transistors (1, 6, 12, 13, 14) formed in the silicon film,
wherein different kinds of single stranded or branched nucleic acid probes (3, 7) are immobilised directly or via a carrier on a second surface of the insulator (17, 29) opposite the first surface to correspond to gates of the insulated gate field effect transistors (1, 6, 12, 13, 14).

3. A DNA microarray according to claim 2, wherein said insulated gate field effect transistors include first (1) and second (6) field effect transistors,
said nucleic acid probes include a nucleic acid probe (3) which has a base sequence complementary to a portion of a target nucleic acid to be detected and is immobilised on the second surface to correspond to a gate of the first insulated gate field effect transistor (1), and a nucleic acid probe (7) which has a base sequence non-complementary to any portion of the target nucleic acid to be detected and is immobilised on said second surface to correspond to a gate of the second insulated gate field effect transistor (6), and
a circuit (9 to 11, 38) is provided to detect and compare outputs of the first and second field effect transistors (1, 6).

4. A method of analyzing nucleic acids comprising the steps of:
introducing a sample solution containing at least one kind of nucleic acid onto said second surface of a DNA microarray according to claim 1 or 2, and subjecting it to hybridization with the single stranded nucleic acid probes or branched nucleic acid probes;
introducing a washing solution (33) onto the second surface, to remove unreacted nucleic acids there from;
introducing an intercalator solution onto the second surface, to react with nucleic acids that have become double stranded;
introducing the washing solution onto the second surface to remove unreacted intercalator there from; and
introducing a buffer onto the second surface to measure outputs of the insulated gate field effect transistors.

5. The method of analyzing nucleic acids according to claim 4, comprising further the steps of:
dissociating the nucleic acids hybridized with the single stranded nucleic acid probes or branched nucleic acid probes by heating the second surface; and
introducing the washing solution onto the second surface to remove the nucleic acids and the intercalator dissociated from the single stranded nucleic acid probes or branched nucleic acid probes, and subsequent repetition of the analysis.

6. A method of analyzing nucleic acids comprising the steps of:
introducing a sample solution containing nucleic acid fragments labeled with a molecule capable of incorporating charged particles onto said second surface of a DNA microarray according to claim 1 or 2, to subject it them to hybridization with the single stranded nucleic acid probes or branched nucleic acid probes;
introducing a washing solution (33) onto the second surface, to remove unreacted nucleic acid fragments there from;
introducing a solution containing an ion to form a complex with the labeling molecule onto the second surface, to react with the molecule labeled on the formed double stranded nucleic acids and form a complex between the ion and the labeling molecule; and
measuring outputs of the insulated gate field effect transistors.

7. The method of analyzing nucleic acids according to claim 6, wherein the molecule capable of incorporating charged particles is a molecule that forms a complex selectively with a monovalent or bivalent cation or anion.

8. The method of analyzing nucleic acids according to claim 6 or 7, wherein plural kinds of molecules capable of incorporating the charged particles are used to label different nucleic acid fragments, respectively, and the charged particles (ions) corresponding to each of the molecules are introduced onto the second surface to allow measurements of plural kinds of genes or genetic polymorphism simultaneously or one after another.

9. The method of analyzing nucleic acids according to any one of claims 6 to 8, further comprising the steps of:
heating the second surface to dissociate the nucleic acid fragments hybridized with the single stranded nucleic acid probes or branched nucleic acid probes; and
introducing the washing solution (33) onto the second surface to remove the nucleic acid fragments dissociated from the single stranded nucleic acid probes or branched nucleic acid probes; and subsequent repetition of the analysis.

10. A process for producing a DNA microarray according to claim 1 or 2, comprising the steps of:
forming a silicon film (19) on said first surface of said insulating substrate (17);
dividing the silicon film into a plurality of silicon film formation areas (19) by patterning the silicon film;
forming a plurality of pn junctions (20) working as source and drain regions (21) of field effect transistors in each of the silicon film formation areas;
carrying out wiring (25) from the source and drain regions with the region between the source and drain regions serving as a channel (24); and
immobilizing said nucleic acid probes (3,7) directly or via a carrier at the sites corresponding to the channels of the field effect transistors on said second surface opposite to the first surface where the silicon film is formed on the insulating substrate.

11. A process for producing a DNA microarray according to claim 1 or 2, comprising the steps of:
forming a silicon film (19) on a surface of an insulating substrate (17);
dividing the silicon film into a plurality of silicon film formation areas (19) by patterning the silicon film;
forming a plurality of pn junctions (20) working as source and drain regions (21) of field effect transistors in each of the silicon film formation areas;
carrying out wiring (25) from the source and drain regions with the region between the source and drain regions serving as a channel (24);
forming an insulating film (18, 29) on the surface where the wiring has been carried out; and
immobilizing nucleic acid probes (3, 7) directly or via a carrier at the sites corresponding to the channels of the field effect transistors on the second surface of the insulating film opposite the first surface of the insulating film which faces the silicon film.

12. A process according to claim 10 or 11, wherein pn junctions (20) working as a heater (22) and a temperature sensor (23) are formed in each of the silicon film formation areas.

13. A DNA microarray according to any of claims 1 to 3, wherein the substantially planar insulator is an insulating substrate (17).

14. A DNA microarray according to any of claims 1 to 3, wherein the substantially planar insulator is an insulating film (29) formed on said silicon film (19) formed on an insulating substrate.

15. A DNA microarray according to 13, wherein said substantially planar insulator (17) has thinned recessed areas (28) at sites corresponding to gates of the insulated gate field effect transistors (1, 6, 12, 13, 14).

16. A DNA microarray according to claim 13, wherein the thickness of the substantially planar insulator is in the range of 0.01 to 1 µm.

## Patentansprüche

1. Potentiometrisches DNA-Mikroarray, aufweisend:
einen im wesentlichen ebenen Isolator (17, 29),
eine auf einer ersten Oberfläche des Isolators ausgebildete Siliziumschicht (19),
einen in der Siliziumschicht ausgebildeten Feldeffekttransistor (1, 6, 12, 13, 14) mit isoliertem Gate, und
eine Bezugselektrode (8),
wobei auf einer der ersten Oberfläche gegenüberliegenden zweiten Oberfläche des Isolators (17, 29) direkt oder über einen Träger eine einsträngige oder verzweigte Nukleinsäuresonde (3, 7) immobilisiert ist.

2. Potentiometrisches DNA-Mikroarray, aufweisend:
einen im wesentlichen planaren Isolator (17, 29),
eine auf einer ersten Oberfläche des Isolators ausgebildete Siliziumschicht (19), und
mehrere in der Siliziumschicht ausgebildete Feldeffekttransistoren (1, 6, 12, 13, 14) mit isoliertem Gate,
wobei auf einer der ersten Oberfläche gegenüberliegenden zweiten Oberfläche des Isolators (17, 20) in Entsprechung zu den Gate-Elektroden der Feldeffekttransistoren (1, 6, 12, 13, 14) mit isoliertem Gate direkt oder über einen Träger verschiedene Arten einsträngiger oder verzweigter Nukleinsäuresonden (3, 7) immobilisiert sind.

3. DNA-Mikroarray nach Anspruch 2, wobei die Feldeffekttransistoren mit isoliertem Gate einen ersten (1) und einen zweiten (6) Feldeffekttransistor beinhalten,
die Nukleinsäuresonden eine Nukleinsäuresonde (3), die eine zu einem Abschnitt einer zu erfassenden Zielnukleinsäure komplementäre Basensequenz aufweist und auf der zweiten Oberfläche in Entsprechung zu einem Gate des ersten Feldeffekttransistors (1) mit isoliertem Gate immobilisiert ist, und eine Nukleinsäuresonde (7) enthalten, die eine zu keinem Abschnitt der zu erfassenden Zielnukleinsäure komplementäre Basensequenz aufweist und auf der zweiten Oberfläche in Entsprechung zu einem Gate des zweiten Feldeffekttransistors (6) mit isoliertem Gate immobilisiert ist, und
eine Schaltung (9 bis 11, 38) zum Erfassen und Vergleichen von Ausgaben des ersten und des zweiten Feldeffekttransistors (1, 6) vorgesehen ist.

4. Verfahren zur Analyse von Nukleinsäuren mit folgenden Schritten:
Aufbringen einer Probenlösung mit mindestens einer Art Nukleinsäure auf die zweite Oberfläche eines DNA-Mikroarrays nach Anspruch 1 oder 2 und hybridisierenlassen mit den einsträngigen Nukleinsäuresonden oder verzweigten Nukleinsäuresonden,
Aufbringen einer Spüllösung (33) auf die zweite Oberfläche, um unreagierte Nukleinsäuren davon zu entfernen,
Aufbringen einer Interkalatorlösung auf die zweite Oberfläche, um mit den doppelsträngig gewordenen Nukleinsäuren zu reagieren,
Aufbringen der Spüllösung auf die zweite Oberfläche, um unreagierten Interkalator davon zu entfernen,
Aufbringen eines Puffers auf die zweite Oberfläche, um Ausgaben der Feldeffekttransistoren mit isoliertem Gate zu messen.

5. Verfahren nach Anspruch 4 mit folgenden weiteren Schritten:
Dissoziieren der mit den einsträngigen Nukleinsäuresonden oder den verzweigten Nukleinsäuresonden hybridisierten Nukleinsäuren durch Erwärmen der zweiten Oberfläche, und
Aufbringen der Spüllösung auf die zweite Oberfläche, um die Nukleinsäuren und den Interkalator, die von den einsträngigen Nukleinsäuresonden oder den verzweigten Nukleinsäuresonden dissoziiert sind, zu entfernen, und nachfolgende Wiederholung der Analyse.

6. Verfahren zur Analyse von Nukleinsäuren mit folgenden Schritten:
Aufbringen einer Probenlösung mit Nukleinsäurefragmenten, die mit einem Molekül markiert sind, das geladene Teilchen aufnehmen kann, auf die zweite Oberfläche eines DNA-Mikroarrays nach Anspruch 1 oder 2, um sie einer Hybridisierung mit den einsträngigen Nukleinsäuresonden oder den verzweigten Nukleinsäuresonden zu unterwerfen,
Aufbringen einer Spüllösung (33) auf die zweite Oberfläche, um unreagierte Nukleinsäurefragmente davon zu entfernen,
Aufbringen einer ein Ion enthaltenden Lösung zur Bildung eines Komplexes mit dem markierenden Molekül auf die zweite Oberfläche, um mit dem markierten Molekül auf den gebildeten doppelsträngigen Nukleinsäuren zu reagieren und zwischen dem Ion und dem markierenden Molekül einen Komplex zu bilden, und
Messen von Ausgaben der Feldeffekttransistoren mit isoliertem Gate.

7. Verfahren nach Anspruch 6, wobei das Molekül, das geladene Teilchen aufnehmen kann, ein Molekül ist, das selektiv mit einem monovalenten oder bivalenten Kation oder Anion einen Komplex bildet.

8. Verfahren nach Anspruch 6 oder 7, wobei verschiedene Arten von Molekülen, die die geladenen Teilchen aufnehmen können, entsprechenderweise zur Markierung verschiedener Nukleinsäurefragmente verwendet werden und die den jeweiligen Molekülen entsprechenden geladenen Teilchen (Ionen) auf die zweite Oberfläche aufgebracht werden, um gleichzeitig oder nacheinander Messungen mehrerer Arten von Genen oder genetischer Polymorphismen zu erlauben.

9. Verfahren nach einem der Ansprüche 6 bis 8, mit folgenden weiteren Schritten:
Erwärmen der zweiten Oberfläche, um die mit den einsträngigen Nukleinsäuresonden oder den verzweigten Nukleinsäuresonden hybridisierten Nukleinsäurefragmente zu dissoziieren, und
Aufbringen einer Spüllösung (33) auf die zweite Oberfläche, um die von den einsträngigen Nukleinsäuresonden oder den verzweigten Nukleinsäuresonden dissoziierten Nukleinsäurefragmente zu entfernen, und nachfolgendes Wiederholen der Analyse.

10. Verfahren zur Herstellung eines DNA-Mikroarrays nach Anspruch 1 oder 2, mit folgenden Schritten:
Ausbilden einer Siliziumschicht (19) auf der ersten Oberfläche des isolierenden Substrats (17),
Teilen der Siliziumschicht in mehrere Siliziumschicht-Ausbildungsbereiche (19) durch Musterbildung der Siliziumschicht,
Ausbilden mehrerer pn-Grenzschichten (20), die als Source- und Drain-Bereiche (21) der Feldeffekttransistoren dienen, in jeder der Siliziumschicht-Ausbildungsbereiche,
Ausführen einer Verdrahtung (25) für die Source- und Drain-Bereiche, wobei der Bereich zwischen den Source- und Drain-Bereichen als Kanal (24) dient, und
Immobilisieren der Nukleinsäuresonden (3, 7) direkt oder über einen Träger an den den Kanälen der Feldeffekttransistoren entsprechenden Orten auf der zweiten Oberfläche, die der ersten Oberfläche gegenüberliegt, auf der die Siliziumschicht auf dem isolierenden Substrat ausgebildet ist.

11. Verfahren zur Herstellung eines DNA-Mikroarrays nach Anspruch 1 oder 2, mit folgenden Schritten:
Ausbilden einer Siliziumschicht (19) auf einer Oberfläche eines isolierenden Substrats (17),
Teilen der Siliziumschicht in mehrere Siliziumschicht-Ausbildungsbereiche (19) durch Musterbildung der Siliziumschicht,
Ausbilden mehrerer pn-Grenzschichten (20), die als Source- und Drain-Bereiche (21) der Feldeffekttransisoren dienen, in jeder der Siliziumschicht-Ausbildungsbereiche,
Ausführen einer Verdrahtung (25) für die Source- und Drain-Bereiche, wobei der Bereich zwischen den Source- und Drain-Bereichen als Kanal (24) dient,
Ausbilden einer Isolierschicht (18, 29) auf der Oberfläche, auf der die Verdrahtung ausgeführt wurde, und
Immobilisieren von Nukleinsäuresonden (3, 7) direkt oder über einen Träger an den Orten, die den Kanälen der Feldeffekttransistoren entsprechen, auf der zweiten Oberfläche der Isolierschicht gegenüber der ersten Oberfläche der Isolierschicht, die der Siliziumschicht zugewandt ist.

12. Verfahren nach Anspruch 10 oder 11, wobei in jeder der Siliziumschicht-Ausbildungsbereiche pn-Grenzschichten (20) ausgebildet werden, die als Heizelement (22) und als Temperatursensor (23) dienen.

13. DNA-Mikroarray nach einem der Ansprüche 1 bis 3, wobei der im wesentlichen ebene Isolator ein isolierendes Substrat (17) ist.

14. DNA-Mikroarray nach einem der Ansprüche 1 bis 3, wobei der im wesentlichen ebene Isolator eine isolierende Schicht (29) ist, die auf der Siliziumschicht (19) ausgebildet ist, die auf einem isolierenden Substrat ausgebildet ist.

15. DNA-Mikroarray nach Anspruch 13, wobei der im wesentlichen ebene Isolator (17) an Orten, die Gate-Elektroden der Feldeffekttransistoren (1, 6, 12, 13, 14) mit isoliertem Gate entsprechen, dünnere vertiefte Bereiche (28) aufweist.

16. DNA-Mikroarray nach Anspruch 13, wobei die Dicke des im wesentlichen ebenen Isolators im Bereich von 0,01 bis 1 µm liegt.

## Revendications

1. Puce à ADN potentiométrique comportant :
un isolant sensiblement plan (17, 29),
un film de silicium (19) formé sur une première surface de l'isolant,
un transistor à effet de champ à grille isolée (1, 6, 12, 13, 14) formé dans le film de silicium, et
une électrode de référence (8),
dans laquelle une sonde d'acide nucléique simple-brin ou ramifiée (3, 7) est immobilisée directement ou via un support sur une seconde surface de l'isolant (17, 29) en vis-à-vis de la première surface.

2. Puce à ADN potentiométrique comportant :
un isolant sensiblement plan (17, 29),
un film de silicium (19) formé sur une première surface de l'isolant, et
une pluralité de transistors à effet de champ à grille isolée (1, 6, 12, 13, 14) formés dans le film de silicium,
dans laquelle différents types de sondes d'acide nucléique simple-brin ou ramifiées (3, 7) sont immobilisés directement ou via un support sur une seconde surface de l'isolant (17, 29) en vis-à-vis de la première surface pour correspondre avec des grilles des transistors à effet de champ à grille isolée (1, 6, 12, 13, 14).

3. Puce à ADN selon la revendication 2, dans laquelle lesdits transistors à effet de champ à grille isolée incluent des premier (1) et second (6) transistors à effet de champ,
lesdites sondes d'acide nucléique incluent une sonde d'acide nucléique (3) qui a une séquence de base complémentaire d'une partie d'un acide nucléique cible à détecter et est immobilisée sur la seconde surface pour correspondre avec une grille du premier transistor à effet de champ à grille isolée (1), et une seconde d'acide nucléique (7) qui a une séquence de base non-complémentaire d'une quelconque partie de l'acide nucléique cible à détecter et est immobilisée sur ladite seconde surface pour correspondre avec une grille du second transistor à effet de champ à grille isolée (6), et
un circuit (9 à 11, 38) est fourni pour détecter et comparer des sorties des premier et second transistors à effet de champ (1, 6).

4. Procédé d'analyse d'acides nucléiques comportant les étapes consistant à :
introduire une solution échantillon contenant au moins un type d'acide nucléique sur ladite seconde surface d'une puce à ADN selon la revendication 1 ou 2, et soumettre celle-ci à une hybridation avec les sondes d'acide nucléique simple-brin ou sondes d'acide nucléique ramifiées,
introduire une solution de lavage (33) sur la seconde surface, pour retirer de celle-ci des acides nucléiques n'ayant pas réagi,
introduire une solution d'intercalation sur la seconde surface, pour réagir avec les acides nucléiques qui sont devenus double-brin,
introduire la solution de lavage sur la seconde surface pour retirer de celle-ci un agent d'intercalation n'ayant pas réagi, et
introduire un tampon sur la seconde surface pour mesurer des sorties des transistors à effet de champ à grille isolée.

5. Procédé d'analyse d'acides nucléiques selon la revendication 4, comportant de plus les étapes consistant à :
dissocier les acides nucléiques hybridés aux sondes d'acide nucléique simple-brin ou sondes d'acide nucléique ramifiées en chauffant la seconde surface, et
introduire la solution de lavage sur la seconde surface pour retirer les acides nucléiques et l'agent d'intercalation dissociés des sondes d'acide nucléique simple-brin ou sondes d'acide nucléique ramifiées, et répétition ultérieure de l'analyse.

6. Procédé d'analyse d'acides nucléiques comportant les étapes consistant à :
introduire un solution échantillon contenant des fragments d'acide nucléique marqués à l'aide d'une molécule capable d'incorporer des particules chargées, sur ladite seconde surface d'une puce à ADN selon la revendication 1 ou 2, pour les soumettre à une hybridation avec les sondes d'acide nucléique simple-brin ou sondes d'acide nucléique ramifiées,
introduire une solution de lavage (33) sur la seconde surface, pour retirer de celle-ci des fragments d'acide nucléique n'ayant pas réagi,
introduire une solution contenant un ion pour former un complexe avec la molécule de marquage sur la seconde surface, pour réagir avec la molécule marquée sur les acides nucléiques double-brin formés et former un complexe entre l'ion et la molécule de marquage, et
mesurer les sorties des transistors à effet de champ à grille isolée.

7. Procédé d'analyse d'acides nucléiques selon la revendication 6, dans lequel la molécule capable d'incorporer des particules chargées est une molécule qui forme de manière sélective un complexe avec un cation ou un anion monovalent ou bivalent.

8. Procédé d'analyse d'acides nucléiques selon la revendication 6 ou 7, dans lequel plusieurs types de molécules capables d'incorporer les particules chargées sont utilisés pour marquer des fragments d'acide nucléique différents, respectivement, et les particules (ions) chargées correspondant à chacune des molécules sont introduites sur la seconde surface pour permettre des mesures de plusieurs types de gènes ou polymorphisme génétique simultanément ou les uns après les autres.

9. Procédé d'analyse d'acides nucléiques selon l'une quelconque des revendications 6 à 8, comportant de plus les étapes consistant à :
chauffer la seconde surface pour dissocier les fragments d'acide nucléique hybridés aux sondes d'acide nucléique simple-brin ou sondes d'acide nucléique ramifiées, et
introduire la solution de lavage (33) sur la seconde surface pour retirer les fragments d'acide nucléique dissociés des sondes d'acide nucléique simple-brin ou sondes d'acide nucléique ramifiées, et répétition ultérieure de l'analyse.

10. Procédé pour produire une puce à ADN selon la revendication 1 ou 2, comportant les étapes consistant à :
former un film de silicium (19) sur ladite première surface dudit substrat isolant (17),
diviser le film de silicium en une pluralité de zones de formation de film de silicium (19) en mettant en forme le film de silicium,
former une pluralité de jonctions pn (20) fonctionnant comme des régions de source et de drain (21) des transistors à effet de champ dans chacune des zones de formation de film de silicium,
effectuer un câblage (25) depuis les régions de source et de drain, la région située entre les régions de source et de drain servant en tant que canal (24), et
immobiliser lesdites sondes d'acide nucléique (3, 7) directement ou via un support au niveau des sites correspondant aux canaux des transistors à effet de champ sur ladite seconde surface en vis-à-vis de la première surface où le film de silicium est formé sur le substrat isolant.

11. Procédé pour produire une puce à ADN selon la revendication 1 ou 2, comportant les étapes consistant à :
former un film de silicium (19) sur une surface d'un substrat isolant (17),
diviser le film de silicium en une pluralité de zones de formation de film de silicium (19) en réalisant un motif sur le film de silicium,
former une pluralité de jonctions pn (20) fonctionnant comme des régions de source et de drain (21) des transistors à effet de champ dans chacune des zones de formation de film de silicium,
effectuer un câblage (25) depuis les régions de source et de drain, la région située entre les régions de source et de drain servant en tant que canal (24),
former un film isolant (18, 29) sur la surface où le câblage a été effectué, et
immobiliser des sondes d'acide nucléique (3, 7) directement ou via un support au niveau des sites correspondant aux canaux des transistors à effet de champ sur la seconde surface du film isolant en vis-à-vis de la première surface du film isolant qui fait face au film de silicium.

12. Procédé selon la revendication 10 ou 11, dans lequel des jonctions pn (20) fonctionnant en tant qu'élément de chauffage (22) et capteur de température (23) sont formées dans chacune des zones de formation de film de silicium.

13. Puce à ADN selon l'une quelconque des revendications 1 à 3, dans laquelle l'isolant sensiblement plan est un substrat isolant (17).

14. Puce à ADN selon l'une quelconque des revendications 1 à 3, dans laquelle l'isolant sensiblement plan est un film isolant (29) formé sur ledit film de silicium (19) formé sur un substrat isolant.

15. Puce à ADN selon la revendication 13, dans laquelle ledit isolant sensiblement plan (17) a des zones évidées amincies (28) à des sites correspondant à des grilles des transistors à effet de champ à grille isolée (1, 6, 12, 13, 14).

16. Puce à ADN selon la revendication 13, dans laquelle l'épaisseur de l'isolant sensiblement plan est dans la plage de 0,01 à 1 µm.
